# EUROPÄISCHE PATENTSCHRIFT

(11) **EP 1 210 929 B1**
(45) Veröffentlichungstag und Bekanntmachung des Hinweises auf die Patenterteilung: **26.04.2006**
(21) Anmeldenummer: 01127510.4
(22) Anmeldetag: 17.11.2001
(51) Int. Cl.: A61K 8/362, A61K 8/37, A61K 8/81, A61Q 17/04, A61Q 19/00, A61K 9/06, A61K 9/107, A61K 47/06

(54) **Kosmetische Mittel enthaltend Alkyl- oder Alkenylbernsteinsäurederivate**
Cosmetic compositions comprising alkyl or alkenyl derivatives of succinic acid
Compositions cosmétiques comprenant des dérivés alkyl ou alkényl de l' acide succinique

(30) Priorität: 29.11.2000 DE 10059318
(43) Veröffentlichungstag der Anmeldung: 05.06.2002
(73) Patentinhaber: Clariant Produkte (Deutschland) GmbH, 65929 Frankfurt am Main (DE)
(72) Erfinder: Klug, Peter, Dr., 63762 Grossostheim (DE); Henning, Torsten, Dr., 65812 Bad Soden (DE); Scherl, Franz Xaver, Dr., 84508 Burgkirchen (DE)

(56) Entgegenhaltungen:
- EP-A- 0 680 946
- EP-A- 1 172 089
- WO-A-02/055046
- US-A- 3 412 111
- US-A- 4 369 123
- US-A- 4 370 253
- US-A- 4 698 065
- US-A- 4 708 753
- US-A- 5 580 553
- CAREY J M ET AL: "IMPROVED HIGH INTERNAL PHASE EMULSIONS USING ALKENYL SUCCINIC ANHYDRIDE BASED EMULSIFIERS" , PROCEEDINGS WORLD SURFACTANTS CONGRESS - COMPTES-RENDUS CONGRES MONDIAL DES AGENTS TENSIOACTIFS - KONGRESSBERICHTE WELT TENSID KONGRESS, XX, XX, VOL. 2, PAGE(S) 883-888 XP002950021 * Seite 1, Absatz 3 * * Seite 2 - Seite 3 * * Abbildungen 1,2 *
- PATENT ABSTRACTS OF JAPAN vol. 012, no. 223 (C-507), 24. Juni 1988 (1988-06-24) & JP 63 020382 A (KAO CORP), 28. Januar 1988 (1988-01-28)
- PATENT ABSTRACTS OF JAPAN vol. 016, no. 470 (C-0990), 30. September 1992 (1992-09-30) & JP 04 169565 A (TOHO CHEM IND CO LTD), 17. Juni 1992 (1992-06-17)

## Beschreibung

Zur Herstellung von kosmetischen Mitteln werden vielfach Kombinationen von Fettsäuresalzen und nichtionischen Tensiden als Emulgatoren eingesetzt. Nachteilig ist dabei die durch die Fettsäuresalze bedingte Steigerung des pH-Wertes, wodurch die kosmetischen Mittel alkalisch reagieren. Da die Oberfläche der menschlichen Haut mit einer schwach sauren Membran (pH 4,5 bis 6,5) bedeckt ist, wird angestrebt, Kosmetika auf einen schwach sauren oder neutralen pH einzustellen. Durch Zugabe von Neutralisationsmittel, wie z.B. Salzsäure, Zitronensäure oder Milchsäure, wird die emulgierende Wirkung der Systeme jedoch beeinträchtigt.

In EP 0 553 241 werden Mischungen aus Alkylpotysacchariden, Fettalkoholen und gegebenenfalls Polysacchariden zur Herstellung von Emulsionen verwendet. In WO 92/07543 werden Alkylglykoside und Fettsäurepartialglyceride als kosmetische Emulgatoren beschrieben.
Da die nichtionischen Tenside eine nur unzureichende Emulgatorwirkung aufweisen, müssen sie in großen Mengen eingesetzt werden, wodurch die Hautfreundlichkeit der kosmetischen Mittel beeinträchtigt wird.
Gute Emulgierwirkung zeigen bekannterfnaßen ethoxylierte Fettsäureester, beispielsweise Poiyethylenglycolstearat (30 EO-Einheiten) und Sorbitanoleate. Ethylenoxidhaltige Produkte stehen jedoch unter dem Verdacht, die Haut für schädliche Stoffe durchlässig zu machen und unter UV-Einwirkung nicht definierte und eventuell schädliche Stoffe zu bilden, weshalb sie für kosmetische Produkte unerwünscht sind.
W/O-Emulsionen enthalten neben emulgierenden Komponenten in der Regel zur Viskositätsstabilisierung Metallseifen und lipophile Wachse, beispielsweise Bienenwachs, Mikrowachse oder Ester von Fettsäuren und Fettalkoholen mit linearen oder verzweigten Fettresten, beispielsweise Stearate, Wollfett oder Wollfettderivate. Nachteilig daran ist, dass derartige Emulsionen aufgrund der zwingend vorhandenen lipophilen Wachse nur in fester Form, d.h. mit einem Schmelz- bzw. Tropfpunkt von 50 bis 60°C, zur Verfügung stehen - oder beim Weglassen der lipophilen Wachse eine unzureichende Phasenstabilität und ein unbefriedigendes Ölbindevermögen aufweisen.

In WO 87/03 613, EP 155 800 und US 4.911.770 werden Carbonsäuren, Carbonsäureanhydride, Ester- sowie Amidderivate mit langkettigen (C₂₀-C₅₀₀)-Alkylresten als Emulgatoren für Explosivstoffe beschrieben.

Überraschend wurde nun gefunden, dass Emulgatoren, umfassend
a) mindestens eine durch Polymerisation von (C₂-C₅) Alkenen erhältliche Alkylkette und/oder Alkenylkette mit mindestens 28 Kohlenstoffatomen, die mit
b) mindestens einer Carbonsäure-, Carbonsäurederivat-, Carbonsäureanhydrid-, Carbonsäureanhydridderivat-, Ester- und/oder Amidgruppe verknüpft ist,
sich besonders gut als Emulgatoren für kosmetische und pharmazeutische Mittel eignen.

Gegenstand der Erfindung sind demnach kosmetische und pharmazeutische Mittel, enthaltend mindestens einen Emulgator, umfassend
a) mindestens eine durch Polymerisation von (C₂-C₅)-Alkenen erhältliche Alkylkette und/oder Alkenylkette mit mindestens 28 Kohlenstoffatomen, die mit
b) mindestens einer Gruppe verknüpft ist, die sich von Maleinsäure und/oder Maleinsäureanhydrid ableitet und durch Umsetzung der Carbonsäure-und/oder Carbonsäureanhydridgruppen mit ethoxylierten und/oder propoxylierten Monoalkoholen, Fettalkoholethoxylaten, Glycerin und/oder Polyglycerinen erhältlich ist.

Als (C₂-C₅)-Alkerie bevorzugt sind Ethylen, Propylen, Buten und Isobuten, besonders bevorzugt Isobuten. Bei den Polyalkylketten und Polyalkylenketten kann es sich auch um Copolymere verschiedener (C₂-C₅)-Alkene handeln.

Als Alkylketten besonders bevorzugt sind Polyisobutenylketten, die durch Polymerisation von Isobuten erhältlich sind.

Die Alkylketten und Alkenylketten a) besitzen bevorzugt 28 bis 200, besonders bevorzugt 40 bis 150, insbesondere bevorzugt 52 bis 100, Kohlenstoffatome.

Als Emulgatoren besonders bevorzugt sind Derivate der Alkenylbernsteinsäureanhydride gemäß Formel (1) 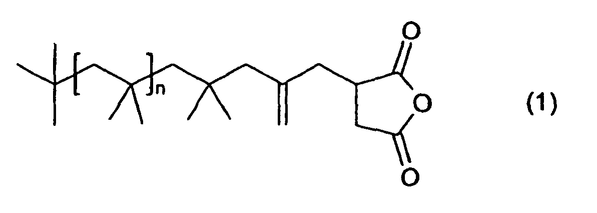 worin n gleich oder größer 4, bevorzugt 4 bis 46, besonders bevorzugt 7 bis 35, insbesondere bevorzugt 10 bis 22, ist. Die Alkenylbernsteinsäureanhydride gemäß Formel (1) können auch in hydrierter Form vorliegen, was sich vorteilhaft auf die Oxidationsstabilität auswirkt.

Die Herstellung der Emulgatoren erfolgt bevorzugt durch En-Reaktion der den Ketten a) entsprechenden, durch Polymerisation der (C₂ - C₅)-Alkene erhältlichen Alkene, mit den den Gruppen b) entsprechenden ungesättigten Carbonsäuren und/oder Carbonsäureanhydride. Gegebenenfalls können die Ketten a) hydriert werden. Die Alkenylbernsteinsäureanhydride gemäß Formel (1) sind bevorzugt durch En-Reaktion der entsprechenden durch Polymerisation von Isobuten erhältlichen Polysiobutene mit Maleinsäureanhydrid erhältlich.
Die Synthese der Emulgatoren kann nach den dem Fachmann geläufigen Techniken erfolgen (s.h. auch WO 87/03613, EP 155 800 und US 4.911.770).

Die Derivatisierung der Carbonsäure- und/oder Carbonsäureanhydridgruppen b) erfolgt vorteilhafterweise im Anschluß an die En-Reaktion. Bevorzugt werden Maleinsäureanhydridgruppen, derivatisiert. Bei der Derivatisierung kann es sich auch um eine gemischte Derivatisierung handeln.

Zur Derivatisierung besonders bevorzugt sind Alk(en)ylbemsteinsäureanhydride gemäß Formel (1).

Für die Derivatisierung mit Alkoholen eignen sich erfindungsgemäß ethoxylierte und/oder propoxylierte Monoalkohole, bevorzugt Methylglykole, insbesondere Methyltriglykol, Ethylglykol und/oder Butylgtykol; Fettalkoholethoxylate; Glycerin; und/oder Polyglycerine.

Als Alkohole besonders bevorzugt sind Methyltriglykol, Glycerin und Polyglycerine.

Bei der Derivatisierung durch Umsetzung mit Alkoholen, beträgt das Molverhältnis von Carbonsäureanhydridgruppen zu Alkohol, bevorzugt 1:0,9 bis 1:2, besonders bevorzugt 1:1. Beim besonders bevorzugten Verhältnis von 1 :1 bilden sich die entsprechenden Halbester

Insbesondere bevorzugt als Emulgatoren sind die durch Umsetzung der Alk(en)ylbemsteinsäureanhydride gemäß Formel (1) mit Glycerin und Methylglykolen, bevorzugt Methyltriglykol, erhältlichen Derivate.

Durch die Derivatisierung können die Emulgiereigenschaften modifiziert und den Anforderungen angepasst werden. Die dann noch freien Carboxylreste können zusätzlich durch Umsetzung mit Aminbasen oder Alkali- und Erdalkalihydroxiden in die entsprechenden Salze überführt werden. Dies ermöglicht die Einstellung eines entsprechenden pH-Wertes in der Endformulierung und modifiziert die Emulgiereigenschaften. Es ist dadurch möglich, geeignete Ernuigatoren für zahlreiche Öle, Fette, Wachse, Paraffine sowie für nicht wassermischbare Lösungsmittel und Wirkstoffe im kosmetischen und pharmazeutischen Sektor bereitzustellen.

Die erfindungsgemäßen Mittel enthalten, bezogen auf die fertigen Mittel, bevorzugt 0,1 bis 8 Gew.-%, besonders bevorzugt 0,3 bis 5 Gew.-%, insbesondere bevorzugt 0,5 bis 4 Gew.-%, an Emulgatoren.

Bei Mitteln handelt es sich bevorzugt um Öl-in-Wasser Emulsionen oder Wasser-in-Öl Emulsionen.

Der nichtwässrige Anteil der Emulsionen, der sich weitgehend aus dem Emulgator und dem Ölkörper zusammensetzt, beträgt bevorzugt 5 bis 95 Gew.-%, besonders bevorzugt 15 bis 75 Gew.-%.
Der Wassergehalt der Emulsionen variiert je nach gewünschter Viskosität der Emulsionen. Lotionen besitzen z.B. eine vergleichsweise niedrige Viskosität, wohingegen Cremes und Salben eine vergleichsweise hohe Viskosität besitzen.

Als Ölkörper eignen sich bevorzugt Guerbetalkohole mit 6 bis 18, vorzugsweise 8 bis 10 Kohlenstoffatomen; Ester von linearen und verzweigten (C₆-C₁₃)-Fettsäuren mit linearen (C₆-C₂₀)-Fettalkoholen; Ester von linearen (C₆-C₁₈)-Fettsäuren mit verzweigten Alkoholen, bevorzugt 2-Ethylhexanol; Ester von linearen und verzweigten Fettsäuren mit mehrwertigen Alkoholen, bevorzugt Dimerdiol und Trimerdiol und/oder Guerbetalkoholen; Triglyceride auf Basis von (C₆-C₁₀)-Fettsäuren; pflanzliche Öle; verzweigte primäre Alkohole; substituierte Cyclohexane; Guerbetcarbonate; Dialkylether; aliphatische Kohlenwasserstoffe, aromatische Kohlenwasserstoffe; Silikonöle und/oder Silikonölderivate.

Als Hilfs- und Zusatzstoffe können die Mittel Co-Emutgatoren, Überfettungsmittel, Fette, Wachse, Stabilisatoren, biogene Wirkstoffe, Lichtschutzstoffe, UV-Lichtschutzfilter, Pigmente, Metalloxide, Mikropigmente, Antioxidantien, Hydrotrope, Solubilisatoren, Konsistenzgeber, Kationpolymere, Glycerin, Konservierungsmittel, Dispergiermittel, Eiweißderivate, wie z.B. Gelatine und Collagenhydrolysate, Polypeptide auf natürlicher und synthetischer Basis, Eigelb, Lecithin, Lanolin; Lanolinderivate, Fettalkohole,, Silicone, deodorierende Mittel, Stoffe mit keratolytischer und keratoplastischer Wirkung, Enzyme, Trägersubstanzen, feuchtigkeitsspendende Stoffe, antimikrobiell wirkende Agentien, Perlglanzmittel, Farb- und/oder Duftstoffe enthalten.

Als nichtionogene O/W-Co-Emulgatoren eignen sich bevorzugt Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; (C₁₂-C₁₈)-Fettsäuremono-und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukte; Anlagerungsprodukte von 15 bis 60 Mol Ethylenoxid an Rizinusöl und/oder gehärtetes Rizinusöl; Polyol-, insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren; Rizinusöl und Alkylphenole, sowie die Glycerinmono- und -diester und Sorbitanmono- und -diester von Fettsäuren stellen bekannte und im Handel erhältliche Produkte dar. Dabei handelt es sich um Homologengemische, deren mittlerer Alkoxylierungsgrad dem Verhältnis der Ausgangsmengen an Ethylenoxid, Propylenoxid und Substrat entspricht. (C₁₂-C₁₈)-Fettsäuremono- und -diester von Anlagerungsprodukten von Ethylenoxid an Glycerin sind aus DE-20 24 051 als Rückfettungsmittel für kosmetische Zubereitungen bekannt.
Als Überfettungsmittel werden bevorzugt polyethoxilierte Lanolinderivate, Lecithinderivate, Polyolfettsäureester, Monoglyceride und Fettsäurealkanolamide verwendet, wobei die letzteren gleichzeitig als Schaumstabilisatoren dienen.

Als Fette bevorzugt sind Glyceride. Als Wachse bevorzugt sind Bienenwachs, Paraffinwachs oder Mikrowachse, gegebenenfalls in Kombination mit hydrophilen Wachsen, wie z.B. Cetylstearylalkohol.

Als Stabilisatoren bevorzugt sind Metallsalze von Fettsäuren, wie z.B. Magnesium-, Aluminium- und/oder Zinkstearat.

Unter biogenen Wirkstoffen sind beispielsweise Pflanzenextrakte und Vitaminkomplexe zu verstehen.

Als Konservierungsmittel eignen sich beispielsweise Phenoxyethanol, Formaldehydlösung, Parabene, Pentandiol oder Sorbinsäure.

Als UV-Filter eignen sich z.B. 4-Aminobenzoesäure, 3-(4'-Trimethylammonium)benzyliden-bornan-2-on-methylsulfat, 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion; 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäureisoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) sowie deren .Natriumsalz und/oder 4-Isopropylbenzyisalicylat.

Als Pigmente/Mikropigmente können z.B. mikrofeines Titandioxid, Zinkoxid und Siliciumoxide eingesetzt werden.

Als Antioxidantien eignen sich beispielsweise Superoxid-Dismutase, Tocopherol (Vitamin E) und Ascorbinsäure (Vitamin C).

Als Farbstoffe können die für kosmetische und pharmazeutische Mittel geeigneten und zugelassenen Substanzen verwendet werden.

Als Verdickungs- und Dispergiermittel besonders geeignet sind Ethylenglykolester von Fettsäuren mit 14 bis 22, besonders bevorzugt 16 bis 22, Kohlenstoffatomen, insbesondere Mono- und Diethylenglykolstearat. Ebenfalls bevorzugt geeignet sind Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-amidobenzoesäure und deren lösliche Salze, N,N-Dihydrocarbyl-(C₁₆-C₁₈)-amidobenzoesäure und deren lösliche Salze und N,N-di(C₁₆-C₁₈)-amidobenzoesäure und deren Derivate. Weiterhin besonders geeignet sind Polyacrylate und Carbomere, insbesondere solche wasserlöslicher oder wasserquellbarer Copolymerisate auf Basis von Acrylamidoalkylsulfonsäuren und N-Vinylcarbonsäureamiden.

Als Solubilisatoren eignen sich prinzipiell alle ein- oder mehrwertigen Alkohole und ethoxylierten Alkohole. Bevorzugt werden Alkohole mit 1 bis 4 Kohlenstoffatomen, wie z.B. Ethanol, Propanol, Isopropanol, n-Butanol und Iso-Butanol, Glycerin und deren Mischungen, eingesetzt. Weiterhin bevorzugt sind Polyethylenglykole mit einer relativen Molekülmasse unter 2000. Insbesondere bevorzugt sind Polyethylenglykole mit einer relativen Molekülmasse zwischen 200 und 600 in Mengen bis zu 45 Gew.-% und Polyethylenglykole mit einer relativen Molekülmasse zwischen 400 und 600 in Mengen von 0,5 bis 15 Gew.-%.
Weitere geeignete Lösungsmittel sind beispielsweise Triacetin (Glycerintriacetat) und 1-Methoxy-2-propanol.

Als Trägermaterialien eignen sich z.B. pflanzliche Öle, natürliche und gehärtete Öle, Wachse, Fette, Wasser, Alkohole, Polyole, Glycerol, Glyceride, flüssige Paraffine, flüssige Fettalkohole, Sterole, Polyethylenglykole, Cellulose und Cellulose-Derivate.

Als fungizide Wirkstoffe eignen sich bevorzugt Ketoconazol, Oxiconazol, Terbinafin, Bifonazole, Butoconazole, Cloconazole, Clotrimazole, Econazole, Enilconazole, Fenticonazole, Isoconazole, Miconazole, Sutconazole, Tioconazole Fluconazole, Itraconazole, Terconazole, Naftifine, Zn-Pyrethion und Octopyrox.

Geeignete kationische Polymere sind z.B. kationische Cellulosederivate; kationische Stärke; Copolymere von Diallylammoniumsalzen und Acrylamiden; quaternierte Vinylpyrrolidon/ Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan. Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane und Polyethersiloxan-Copolymere - wie in US 5104 645 und den darin zitierten Schriften beschrieben - die bei Raumtemperatur sowohl flüssig als auch harzförmig vorliegen können.

Die erfindurigsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen abgemischt werden.

Als feuchtigkeitsspendende Substanz stehen beispielsweise Isopropylpalmitat, Glycerin und/ oder Sorbitol zu Verfügung, die bevorzugt in Mengen von 0,1 bis 50 Gew.-%, eingesetzt werden.

Als deodorierende Stoffe können z.B. Allantoin und Bisabolol, bevorzugt in Mengen von 0,0001 bis 10 Gew.% eingesetzt werden.

Als Perlglanzmittel kommen beispielsweise Glycoldistearinsäureester, wie z.B. Ethylenglycoldistearat, aber auch Fettsäuremonoglykolester in Betracht.

Der Gesamtanteil der Hilfs- und Zusatzstoffe beträgt, bezogen auf die fertigen Mittel, bevorzugt 1 bis 10 Gew.-%, besonders bevorzugt 2 bis 5 Gew.-%.

Im Falle erfindungsgemäßer Emulsionen kann die Herstellung in an sich bekannter Weise, d.h. beispielsweise durch Heiß-, Kalt-, Heiß-Heiß/Kalt- bzw. PIT-Emulgierung, erfolgen.

Die erfindungsgemäßen Mittel zeichnen sich durch eine gute Wärme- und Kältestabilität, hohe Wasseraufnahmefähigkeit bei gleichzeitig hohem Ölbindevermögen, gute Hautverträglichkeit und gute Verträglichkeit mit den in kosmetischen und pharmazeutischen Mitteln gebräuchlichen Inhaltsstoffen aus. Besonders vorteilhaft ist, dass die erfindungsgemäßen Mittel frei sein können von lipophilen Wachsen, wie z.B. Bienenwachs, Mikrowachsen, hydriertem Rizinusöl oder Estern von Fettsäuren und Fettalkoholen mit linearen oder verzweigten Fettresten, wie z.B. Stearaten, Wollfetten und Wollfettderivaten, und dennoch eine hohe Viskositätsstabilität erreicht wird. Ein weiterer Vorteil ist die hohe Stabilität der erfindungsgemäßen Mittel, die ansonsten nur mit Hilfe von eher hochpreisigen Silikonemulgatoren erreicht werden kann.
Bei den kosmetischen und pharmazeutischen Mitteln kann es sich z.B. um Hautpflegemittel, wie z.B. Tagescremes, Nachtcremes, Pflegecremes, Nährcremes und Bodylotions; pharmazeutische Wirkstoffe enthaltende Salben, Cremes und Lotionen; Deo-Sprays; Deo-Sticks; Deo-Gele; dekorierende Kosmetik; Sonnenschutzmittel; After-Sun Lotions und Lotionen für die Herstellung von feuchtigkeitsgetränkten Reinigungs- und Pflegetüchem handeln.

Die nachfolgenden Beispiele dienen der näheren Erläuterung der Erfindung, ohne sie jedoch darauf einzuschränken.

### Herstellbeispiele:

Als Basisemulgator für die nachfolgenden Beispiele wurde ein Polyisobutenylbernsteinsäureanhydrid mit einem Gebrauchsmoi von 918,3 g/mol verwendet. Dieses wurde durch thermische Umsetzung von Polyisobuten ®Glissopal 1000 (BASF) mit einem Überschuss von 1,5 Moläquivalenten Maleinsäureanhydrid bei 205 °C hergestellt und durch Vakuumdestillation vom überschüssigen Maleinsäureanhydrid befreit. Es stellt bei Raumtemperatur ein hochviskoses, zähes Öl dar.

### Herstellbeispiel 1:

Ester aus Polyisobutenylbernsteinsäureanhydrid und Glycerin 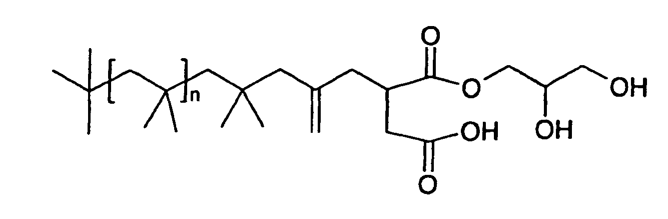 204,6 g eines Polyisobutenylbernsteinsäureanhydrids mit einem Gebrauchsmol von 918,3 g/mol, 87,7 g Paraffinöl (niedrigviskos) und 20,5 g Glycerin wurden unter Stickstoffatmosphäre vorgelegt, auf 100°C erhitzt und 5 Stunden bei dieser Temperatur gerührt. Anschließend wurde das Produkt abgekühlt. Es entstand ein hellgelbes Öl mit einer Säurezahl von 37,4 mg KOH/g.

### Herstellbeispiel 2:

Ester aus Polyisobutenylbernsteinsäureanhydrid und Methyltriglykol 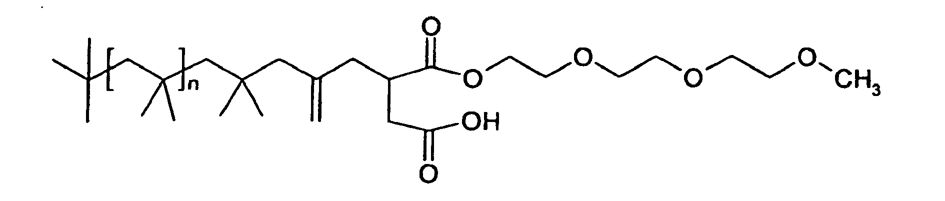 206,1 g eines Polyisobutenylbernsteinsäureanhydrids mit einem Gebrauchsmol von 918,3 g/mol, 88,4 g Paraffinöl (niedrigviskos) und 36,1 g Methyltriglykol wurden unter Stickstoffatmosphäre vorgelegt, auf 100°C erhitzt und 5 Stunden bei dieser Temperatur gerührt. Anschließend wurde das Produkt abgekühlt. Es entstand ein hellgelbes ÖI mit einer Säurezahl von 37,8 mg KOH/g.

### Anwendungsbeispiele (bei den Prozentangaben handelt es sich um Gew. %)

### Beispiel 1: W/O Nachtcreme (ohne Stabilisatoren wie z.B. Wachse oder Stearate)

| | |
|---|---|
| Phase A | |
| Emulgator aus Herstellbeispiel 1 | 1,00 % |
| Hostacerin DGI (Polyglyceryl-2 Sesquiisostearate) | 1,00 % |
| Isododecan | 3,00 % |
| Isohexadecan | 5,00 % |
| Paraffinöl, niedrigviskos | 6,25 % |
| Isopropylpalmitat | 3,75 % |
| Sojaöl | 2,50 % |
| Phase B | |
| Wasser | ad 100 % |
| Natriumchlorid | 0,60 % |
| Glycerin | 1,00 % |

Die Ölphase A wird auf 80°C erhitzt, dann wird die Wasserphase B unter Rühren mit einem hochdispergierenden Werkzeug zugegeben. Die Emulsion ist bei einer Lagertemperatur von +50 °C mindestens 6 Wochen stabil. Die Emulsion ist nach 5 Zyklen im Schaukeltest (-12°C / +50°C) stabil.

### Beispiel 2: W/O Softcreme (kalt herstellbar, ohne Stabilisatoren wie z.B. Wachse oder Stearate)

| | |
|---|---|
| Phase A | |
| Emulgator aus Herstellbeispiel 2 | 2,00 % |
| Hostacerin DGI (Polyglyceryl-2 Sesquiisostearate) | 1,00 % |
| Paraffinöl, niedrigviskos | 14,00 % |
| Isopropylpalmitat | 5,00 % |
| Octyldodecanol | 5,00 % |
| Phase B | |
| Wasser | ad 100,00 % |
| Natriumchlorid | 2,00 % |

Die Wasserphase B wird bei Raumtemperatur unter Rühren mit einem hochdispergierenden Werkzeug der Ölphase A zugegeben. Die Emulsion ist bei einer Lagertemperatur von +50°C mindestens 6 Wochen stabil. Die Emulsion ist nach 5 Zyklen im Schaukeltest (-12°C / +50°C) stabil.

### Beispiel 3: W/O Sonnenschutzcreme (ohne Stabilisatoren wie z.B. Wachse oder Stearate)

| Phase A | |
|---|---|
| Emulgator aus Herstellbeispiel 1 | 2,00 % |
| Isohexadecan | 5,00 % |
| Isododecan | 3,00 % |
| Paraffinöl, niedrigviskos | 6,25 % |
| Isopropylpalmitat | 3,75 % |
| Isoamyl p-Methoxycinnamate | 4,00 % |
| Benzophenone-3 | 1,00 % |

| Phase B | |
|---|---|
| Wasser | ad 100 % |
| Natriumchlorid | 0,60 % |
| Glycerin | 1,00 % |

Die Ölphase A wird auf 80°C erhitzt, dann die Wasserphase B unter Rühren mit einem hochdispergierenden Werkzeug zugegeben. Die Emulsion ist bei einer Lagertemperatur von +50°C mindestens 6 Wochen stabil. Die Emulsion ist nach 5 Zyklen im Schaukeltest (-12°C / +50°C) stabil.

### Beispiel 4: W/O Babycreme (ohne Stabilisatoren wie z.B. Wachse oder Stearate)

| Phase A | |
|---|---|
| Emulgator aus Herstellbeispiel 1 | 2,00 % |
| Hostacerin DGI (Polyglyceryl-2 Sesquiisostearate) | 1,00 % |
| Isododecan | 3,00 % |
| Isohexadecan | 5,00 % |
| Paraffinöl, niedrigviskos | 6,25 % |
| Isopropylpalmitat | 3,75 % |

| Phase B | |
|---|---|
| Talkum | 10,00 % |
| Zinkoxid | 10,00 % |

| Phase C | |
|---|---|
| Wasser | ad 100 % |
| Natriumchlorid | 0,60 % |
| Glycerin | 1,00 % |

Die Ölphase A wird auf 80°C erhitzt, dann Komponenten B zugeben. Die Wasserphase C wird unter Rühren mit einem hochdispergierenden Werkzeug zugegeben. Die Emulsion ist bei einer Lagertemperatur von +50°C mindestens 6 Wochen stabil. Die Emulsion ist nach 5 Zyklen im Schaukeltest (-12°C / +50°C) stabil.

## Patentansprüche

1. Kosmetische und pharmazeutische Mittel, enthaltend mindestens einen Emulgator, umfassend
a) mindestens eine durch Polymerisation von (C₂-C₅)-Alkenen erhältliche Alkylkette und/oder Alkenylkette mit mindestens 28 Kohlenstoffatomen, die mit
b) mindestens einer Gruppe verknüpft ist, die sich von Maleinsäure und/oder Maleinsäureanhydrid ableitet und durch Umsetzung der Carbonsäure-und/oder Carbonsäureanhydridgruppen mit ethoxylierten und/oder propoxylierten Monoalkoholen; Fettalkoholethoxylaten; Glycerin und/oder Polyglycerinen erhältlich ist.

2. Mittel nach Anspruch 1, **dadurch gekennzeichnet, dass** es sich bei den (C₂-C₅)-Alkenen um Ethylen, Propylen, Buten und/oder Isobuten handelt.

3. Mittel nach Anspruch 1 und/oder 2, **dadurch gekennzeichnet, dass** es sich bei den Ketten a) um durch Polymerisation von Isobuten erhältliche Polyisobutenylketten handelt.

4. Mittel nach mindestens einem der Ansprüche 1 bis 3, **dadurch gekennzeichnet, dass** die Alkylketten und Alkenylketten a) 28 bis 200 Kohlenstoffatome besitzen.

5. Mittel nach mindestens einem der Ansprüche 1 bis 4, **dadurch gekennzeichnet, dass** es sich bei den Emulgatoren um Derivate von Alkenylbernsteinsäureanhydriden gemäß Formel (1) handelt, 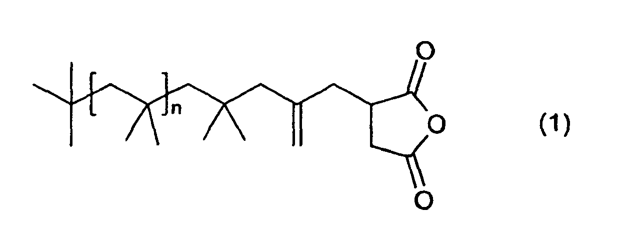 wobei n gleich oder größer 4 ist.

6. Mittel nach mindestens einem der Ansprüche 1 bis 4, **dadurch gekennzeichnet, dass** es sich bei den unter b) genannten Alkoholen um Methylglykole; Glycerin und/oder Polyglycerine handelt.

7. Mittel nach Anspruch 5, **dadurch gekennzeichnet, dass** es sich bei den Emulgatoren um solche handelt, die durch Umsetzung von Alk(en)ylbernsteinsäureanhydriden gemäß Formel (1) mit Glycerin und/oder Methylglykolen erhältlich sind.

8. Mittel nach Anspruch 7, **dadurch gekennzeichnet, dass** es sich bei den Emulgatoren um solche handelt, die durch Umsetzung von Alk(en)ylbemsteinsäureanhydriden gemäß Formel (1) mit Glycerin und/oder Methyltriglykol erhältlich sind.

9. Mittel nach mindestens einem der Ansprüche 1 bis 8, **dadurch gekennzeichnet, dass** bei der Umsetzung mit Alkoholen das Molverhältnis von Carbonsäureanhydridgruppen zu Alkohol 1: 0,9 bis 1 : 2, bevorzugt 1:1, beträgt.

10. Mittel nach mindestens einem der Ansprüche 1 bis 9, **dadurch gekennzeichnet, dass** sie, bezogen auf die fertigen Mittel, 0,1 bis 8 Gew.-% an Emulgatoren enthalten.

11. Mittel nach mindestens einem der Ansprüche 1 bis 10, **dadurch gekennzeichnet, daß** es sich dabei um Emulsionen handelt.

12. Mittel nach mindestens einem der Ansprüche 1 bis 11, **dadurch gekennzeichnet, dass** es sich dabei um Salben, Cremes, Lotionen, Gele oder Sprays handelt.

## Claims

1. A cosmetic or pharmaceutical composition containing at least one emulsifier comprising
a) at least one alkyl chain and/or alkenyl chain having at least 28 carbon atoms obtainable by polymerization of (C₂-C₅)-alkenes and which is linked with
b) at least one group which is derived from maleic acid and/or maleic anhydride and is obtainable by reacting the carboxylic acid and/or carboxylic anhydride groups with ethoxylated and/or propoxylated monoalcohols; fatty alcohol ethoxylates; glycerol and/or polyglycerols.

2. The composition as claimed in claim 1, wherein the (C₂-C₅)-alkenes are ethylene, propylene, butene and/or isobutene.

3. The composition as claimed in claim 1 and/or 2, wherein the chains a) are polyisobutenyl chains obtainable by polymerization of isobutene.

4. The composition as claimed in at least one of claims 1 to 3, wherein the alkyl chains and alkenyl chains a) have 28 to 200 carbon atoms.

5. The composition as claimed in at least one of claims 1 to 4, wherein the emulsifiers are derivatives of alkenylsuccinic anhydrides according to formula (1), 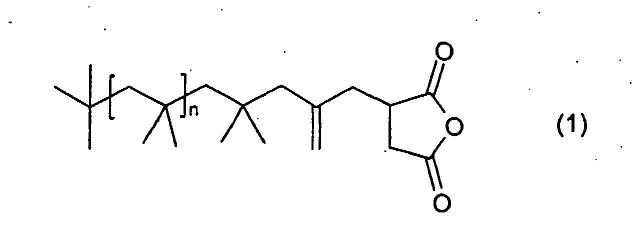 where n is equal to or greater than 4.

6. The composition as claimed in at least one of claims 1 to 4, wherein the alcohols specified under b) are methyl glycols; glycerol and/or polyglycerols.

7. The composition as claimed in claim 5, wherein the emulsifiers are those obtainable by reacting alk(en)ylsuccinic anhydrides according to formula (1) with glycerol and/or methyl glycols.

8. The composition as claimed in claim 7, wherein the emsulsifiers are those obtainable by reacting alk(en)ylsuccinic anhydrides according to formula (1) with glycerol and/or methyl triglycol.

9. The composition as claimed in at least one of claims 1 to 8, wherein, in the reaction with alcohols, the molar ratio of carboxylic anhydride groups to alcohol is 1:0.9 to 1:2, preferably 1:1.

10. The composition as claimed in at least one of claims 1 to 9, wherein, based on the finished composition, 0.1 to 8% by weight of emulsifiers are present.

11. The composition as claimed in at least one of claims 1 to 10, which is in the form of an emulsion.

12. The composition as claimed in at least one of claims 1 to 11, which is in the form of an ointment, cream, lotion, gel or spray.

## Revendications

1. Composition cosmétique et pharmaceutique, contenant au moins un agent émulsionnant, comprenant
a) au moins une chaîne alkyle et/ou une chaîne alcényle avec au moins 28 atomes de carbone, pouvant être obtenue par polymérisation d'alcènes en C₂ à C₅, qui
b) est reliée au moins à un groupe, qui dérive de l'acide maléique et/ou de l'anhydride d'acide maléique et peut être obtenu par réaction de groupes acide carboxylique et/ou anhydride d'acide carboxylique avec des monoalcools éthoxylés et/ou propoxylés ; des éthoxylates d'alcool gras ; le glycérol et/ou des polyglycérols.

2. Composition selon la revendication 1, **caractérisée en ce qu'**il s'agit pour les alcènes en C₂ à C₅ de l'éthylène, propylène, butène et/ou isobutène.

3. Composition selon la revendication 1 et/ou 2, **caractérisée en ce qu'**il s'agit pour les chaînes a) de chaînes polyisobutényle pouvant être obtenues par polymérisation d'isobutène.

4. Composition selon au moins une des revendications 1 à 3, **caractérisée en ce que** les chaînes alkyle et les chaînes alcényle a) possèdent 28 à 200 atomes de carbone.

5. Composition selon au moins une des revendications 1 à 4, **caractérisée en ce qu'**il s'agit pour les agents émulsionnants de dérivés d'anhydrides d'acide alcénylsuccinique selon la formule (1), 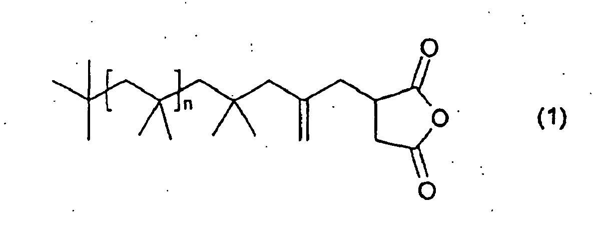 dans laquelle n est égal ou supérieur à 4.

6. Composition selon au moins une des revendications 1 à 4, **caractérisée en ce qu'**il s'agit pour les alcools mentionnés en b) de méthylglycols ; glycérol et/ou polyglycérols.

7. Composition selon la revendication 5, **caractérisée en ce qu'**il s'agit pour les agents émulsionnants de ceux qui peuvent être obtenus par réaction d'anhydrides d'acide alkyl- ou alcénylsuccinique selon la formule (1) avec le glycérol et/ou des méthylglycols.

8. Composition selon la revendication 7, **caractérisées en ce qu'**il s'agit*de ceux qui peuvent être obtenues par réaction d'anhydrides d'acide alkyl-ou alcénylsuccinique selon la formule (1) avec le glycérol et/ou le méthyl-triglycol.
*pour les agents émulsionnants

9. Composition selon au moins une des revendications 1 à 8, **caractérisée en ce que** dans la réaction avec des alcools le rapport en moles des groupes anhydride d'acide carboxylique à l'alcool est de 1 : 0,9 à 1 : 2, de préférence 1 : 1.

10. Composition selon au moins une des revendications 1 à 9, **caractérisée en ce qu'**elle contient, par rapport à la composition prête à l'emploi, 0, 1 à 8 % en poids d'agents émulsionnants.

11. Composition selon au moins une des revendications 1 à 10, **caractérisée en ce qu'**il s'agit d'émulsions.

12. Composition selon au moins une des revendications 1 à 11, **caractérisée en ce qu'**il s'agit d'onguents, de crèmes, de lotions, de gels ou d'aérosols.
